(19) Europäisches Patentamt
European Patent Office
Office européen des brevets

(11) Numéro de publication : **0 301 925 B1**

(12) # FASCICULE DE BREVET EUROPEEN

(45) Date de publication du fascicule du brevet : **23.10.91 Bulletin 91/43**

(51) Int. Cl.$^5$ : **C07C 211/27,** C07C 211/29, C07C 209/66, C07C 215/28

(21) Numéro de dépôt : **88401067.9**

(22) Date de dépôt : **03.05.88**

(54) **Nouveau procédé de préparation énantiospécifique du (S) éthylamino-2 (trifluorométhyl-3 phényl)-1 propane.**

(30) Priorité : **16.07.87 FR 8710020**

(43) Date de publication de la demande : **01.02.89 Bulletin 89/05**

(45) Mention de la délivrance du brevet : **23.10.91 Bulletin 91/43**

(84) Etats contractants désignés : **AT BE CH DE FR GB IT LI LU NL SE**

(56) Documents cités :
**CH-A- 204 708**
**FR-M- 5 566**
**US-A- 3 792 048**

(73) Titulaire : **ADIR ET COMPAGNIE**
**1 rue Carle Hébert**
**F-92415 Courbevoie Cédex (FR)**

(72) Inventeur : **Fugier, Claude**
**34 Rue André Caplet**
**F-76600 Le Havre (FR)**
Inventeur : **Leroux, Michel**
**Lot No 1, Nointot**
**F-76210 Bolbec (FR)**
Inventeur : **Mauge, Robert**
**7 Rue Edouard Herriot**
**F-76600 Le Havre (FR)**
Inventeur : **Alexakis, Alexandre**
**11 Rue Charbonnel**
**F-75013 Paris (FR)**
Inventeur : **Normant, Jean-François**
**9 Rue Paul Doumer**
**F-92340 Bourg La Reine (FR)**

**EP 0 301 925 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau procédé de préparation énantiospécifique du (S) éthylamino-2 (trifluorométhyl-3 phényl)-1 propane.

Ce composé possède des propriétés pharmacologiques très intéressantes et notamment des propriétés anorexigènes très puissantes sans, toutefois, présenter les effets indésirables des dérivés amphétaminiques connus (Goodman et Gilman, The Pharmacological Basis of Therapeutics, 7e Ed, Mac Millan Ed. N.Y., 1985 p.178).

Selon le brevet initial (FR N°1.324.220), le composé de formule I est obtenu sous sa forme racémique à partir de l'amino-2 (trifluorométhyl-3 phényl)-1 propane racémique dont plusieurs voies de synthèses sont proposées. Plus récemment, le brevet DD N°108.971 a également décrit une nouvelle voie de synthèse du composé de formule I sous forme racémique.

Cependant, il est connu que l'éthylamino-2 (trifluorométhyl-3 phényl)-1 propane racémique est très résistant aux procédés habituels de résolution (Brevet GB N°814.339). Le brevet français N°1.468.724 décrit un procédé efficace de séparation du racémique par action de l'acide d-camphorique pour obtenir le (S) éthylamino-2 (trifluorméthyl-3 phényl)-1, propane dextrogyre. Cependant, quelle que soit l'efficacité du procédé de séparation, on est toujours confronté à un double problème. D'une part, le nombre important d'opérations nécessitées par la séparation qui complique la préparation industrielle et d'autre part, le coût de préparation de ce composé est trop élevé vu que son isomère lévogyre ne présente pas le même intérêt pharmacologique.

La demanderesse a maintenant découvert un nouveau procédé de préparation énantiospécifique du (S) éthylamino-2 (trifluorométhyl-3 phényl)-1 propane très avantageux en regard des procédés connus qui exigent le dédoublement du racémique.

L'invention a plus précisément pour objet un procédé de préparation énantiospécifique du (S) éthylamino-2 (trifluorométhyl-3 phényl)-1 propane de formule (I),

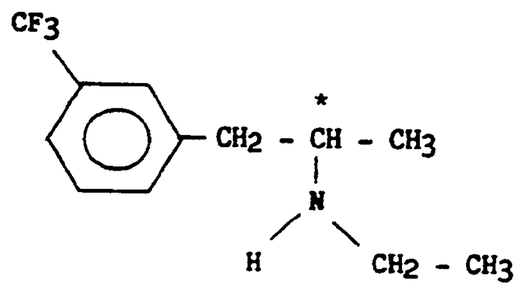

(S) (I)

caractérisé en ce que :

— l'on condense le (S) alaninol ou (S) amino-2 propanol-1, composé de formule II :

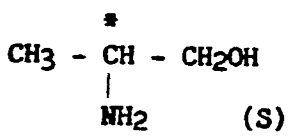

(II)

avec le benzaldéhyde, à température ambiante, et dans un solvant alcoolique anhydre, et ensuite, que l'on réduit à l'aide d'un borohydrure de métal alcalin pour obtenir le (S) N-benzyl alaninol ou (S) benzylamino-2 propanol-1 composé de formule III :

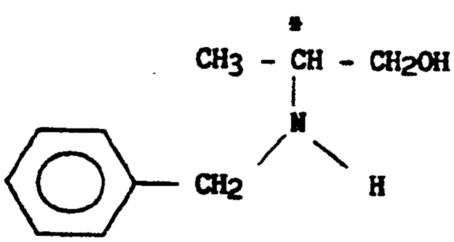

(S) (III)

que l'on fait réagir avec l'acétaldéhyde dans le même milieu réactionnel, pour obtenir après réduction par un borohydrure de métal alcalin, le (S) N-benzyl, N-éthyl alaninol, ou (S) (N-benzyl N-éthyl amino)-2 propanol-1, composé de formule IV :

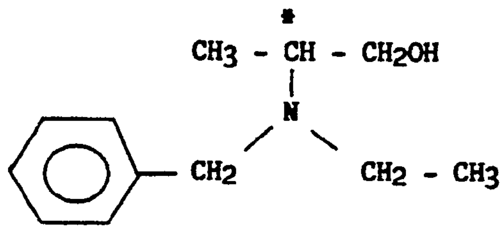

(IV)

(S)

que l'on soumet à l'action du chlorure de thionyle en solution dans l'éther et en présence d'un solvant aprotique, pour former le chlorhydrate du (S) (N-benzyl N-éthyl amino)-2 chloro-1 propane, composé de formule V :

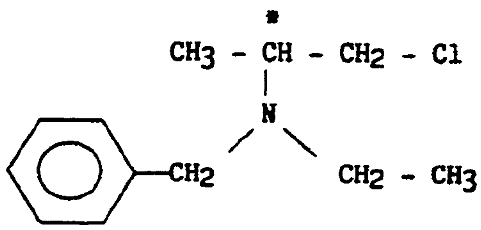

(V)

, HCl

(S)

dont on libère la base par action d'un sel minéral alcalin tel que le carbonate de sodium,
que l'on condense en présence d'un halogénure de cuivre, dans un solvant organique inerte, avec un composé organométallique de formule VI :

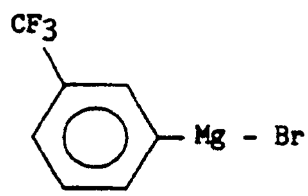

(VI)

pour former, après hydrolyse basique, le (S) (N-benzyl N-éthyl amino)-2 (trifluorométhyl-3 phényl)-1, propane composé de formule VII :

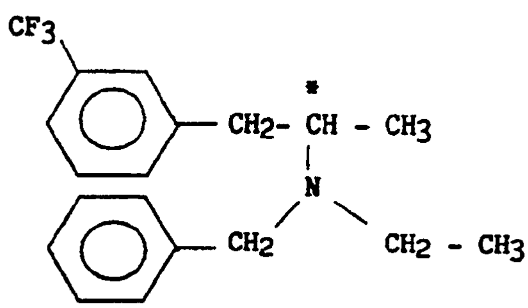

(VII)

que l'on soumet à une hydrogénation catalytique sous pression, en présence d'un métal de transition et à une température comprise entre 20°C et 60°C pour obtenir le (S) éthylamino-2 (trifluorométhyl-3 phényl)-1 propane de formule I.

Selon le procédé de l'invention, la condensation du (S) alaninol composé de formule II avec le benzaldéhyde et du (S) N-benzyl alaninol composé de formule III avec l'acétaldéhyde s'effectue dans un solvant alcoolique anhydre, de préférence le méthanol, à une température comprise entre 0°C et +10°C.

Pour la réduction des produits de condensation dy benzaldéhyde sur le (S) alaninol et de l'acétaldéhyde

sur le (S) N-benzyl alaninol, on préfère utiliser le borohydrure de sodium.

La réaction du (S) N-benzyl, N-éthyl alaninol, composé de formule IV, avec le chlorure de thionyle s'effectue dans un solvant organique inerte de préférence un éther en présence d'un solvant aprotique comme l'hexaméthyle triamide phosphore (HMPT), à une température comprise entre −10°C et 20°C.

La condensation du composé organométallique de formule VI sur le (S) (N-benzyl N-éthyl amino)-2 chloro-1 propane s'effectue en présence d'un catalyseur choisi parmi les halogénures de cuivre (I), dans un solvant organique inerte comme un éther ou de préférence le tétrahydrofuranne, à une température comprise entre −20° et 10°C. Le produit de la réaction est hydrolysé d'abord par une solution saturée de chlorure d'ammonium puis par une solution concentrée d'ammoniaque.

L'hydrogénolyse du composé VII est effectuée par l'hydrogène sous une pression comprise entre 5 et 15 bars, à une température comprise entre 20° et 60°C en présence d'un catalyseur choisi parmi les métaux de transition, par exemple le palladium à 5% sur charbon.

Le composé de formule III est décrit dans la littérature sous sa forme racémique (J. Med. Chem., 1971, 14, N°7, p. 104) mais l'énantiomère (S) n'a jamais été décrit. Les composés IV, V et VII sont nouveaux et à ce titre font aussi partie de l'invention.

Le (S) alaninol utilisé comme produit de départ, ainsi que le benzaldéhyde et l'acétaldéhyde utilisés comme réactifs sont des produits disponibles dans le commerce. La préparation du composé VI est connue (Marvel C.S., Overberger C.G., Alley R.E., Saunders J.H., J. Am. Chem., (1946), 68, p. 736-738).

Le procédé décrit ci-dessus, objet de la présente invention, permet d'obtenir avec de bons rendements, le (S) éthylamino-2 (trifluorométhyl-3 phényl)-1 propane identique à l'énantiomère (S) obtenu par dédoublement du racémique du (R, S) éthylamino-2 (trifluorométhyl-3 phényl)-1 propane.

La nouveauté de l'invention réside dans le choix particulier des produits de départ simples qui permettent un procédé particulièrement avantageux et qui, surtout, permettent d'obtenir sélectivement l'énantiomère (S) évitant ainsi la délicate opération de dédoublement du racémique qui entraîne inévitablement une perte importante de produit.

La présente invention est illustrée de façon plus détaillée par les exemples ci-après :

Les spectres de résonance magnétique nucléaire du carbone ($^{13}C$) ont été enregistrés à 50 MHz en solution dans le deuterochloroforme.

**EXEMPLE 1 :**

**(S) N-benzyl N-éthyl, alaninol**

Dans un bécher muni d'une agitation magnétique, charger 30 g de (S) alaninol et 400 ml de méthanol pur anhydre. Couler rapidement 42,4 g de benzaldéhyde en solution dans 200 ml de méthanol anhydre. Poursuivre l'agitation pendant 1 heure à température ambiante. Refroidir le milieu réactionnel par un bain d'eau glacée et additionner en 20 minutes sans dépasser 20°C, 7,6 g de borohydrure de sodium. Agiter pendant 30 minutes à température ambiante. Après refroidissement, par un bain d'eau glacée, additionner sans dépasser 17°C, 35,2 g d'acétaldéhyde. Maintenir l'agitation pendant 30 minutes à 17°C puis refroidir à nouveau par de l'eau glacée et ajouter 11,4 g de borohydrure de sodium en 30 minutes sans dépasser+17°C. Agiter à température ambiante pendant 15 heures, puis ajouter 25 ml d'une solution saturée de chlorure de sodium et 325 ml de dichlorométhane. Agiter, il se forme un précipité blanc qui est filtré après 15 minutes d'agitation. Le filtrat est évaporé à 50°C sous une pression de 13 mmHg. Reprendre le résidu d'évaporation par un mélange contenant 500 ml de dichlorométhane, 50 ml de solution saturée de chlorure de sodium et 50 ml d'eau. Agiter pendant 30 minutes puis décanter. Extraire la phase aqueuse par 50 ml de dichlorométhane. Laver la phase organique par 25 ml d'eau et sécher sur carbonate de sodium anhydre. Evaporer le solvant à 50°C sous une pression de 13 mmHg. On obtient 73,7 g de produit brut. Après distillation à 120°C sous 0.2 mmHg, on obtient 64,5 g de (S) N-benzyl, N-éthyl alaninol.

La pureté du produit est supérieure à 95%. Elle a été déterminée par chromatographie en phase gazeuse, sur colonne capillaire SE-52, de 25 m de long, en isotherme de température à 220°C.

Rendement : 83.5%

$\alpha_D$ : +84.06°

**EXAMPLE 2 :**

**(S) (N-benzyl N-éthyl amino)-2, chloro-1 propane**

Dans un ballon tricol muni d'une agitation et placé sous atmosphère d'azote, charger 19,3 g de (S) N-benzyl, N-éthyl alaninol, 350 ml d'éther éthylique anhydre puis 60 ml d'hexaméthyle triamide phosphore (HMPT).

A l'aide d'une saumure, refroidir le milieu réactionnel à –5°C et couler 13 g de chlorure de thionyle dilué dans 100 ml d'éther en restant à la même température. Un précipité beige se forme. Retirer le bain réfrigérant et poursuivre l'agitation pendant 15 heures avec remontée progressive à température ambiante. Eliminer sous vide et sous agitation, l'anhydride sulfureux formé.

Le chlorhydrate du (S) (N-benzyl N-éthyl amino)-2, chloro-1 propane formé se trouve en suspension dans l'éther. Couler 157 ml d'une solution saturée de carbonate de sodium afin de libérer la base. Le pH du milieu réactionnel est alors égal à 6,5. Décanter, puis laver la couche organique par deux fois 25 ml d'eau pour éliminer les traces d'HMPT. Après séchage sur sulfate de magnésium évaporer le solvant. On obtient 21,4 g de produit brut que l'on distille sous vide (Eb 94-96°C/0,5 mmHg). On obtient 18,16 g de (N-benzyl N-éthyl amino)-2 chloro-1 propane.

La pureté du produit est supérieure à 96%. Elle a été déterminée par chromatographie en phase gazeuse sur colonne capillaire SE-52 de 25 m de long en isotherme de température à 180°C.

Rendement : 85,9%

$\alpha_D$ = +32,39°

**EXAMPLE 3 :**

**(S) (N-benzyl N-éthyl amino)-2, (trifluorométhyl -3 phényl)-1 propane**

Dans un ballon tricol de 500 ml muni d'un agitateur, charger 15,86 g de (S) (N-benzyl N-éthyl amino)-2, chloro-1 propane, 200 ml de tétrahydrofuranne anhydre et 1,6 g d'iodure cuivreux. Après refroidissement du milieu réactionnel à –15°C, couler 100 ml d'une solution molaire de bromure de trifluorométhyl-3 phényl magnésium. Porter à reflux pendant environ 3 heures. Refroidir le milieu réactionnel dans le tétrahydrofuranne à –10°C, puis hydrolyser par 100 ml d'une solution saturée de chlorure d'ammonium puis par 100 ml d'ammoniaque concentrée. Poursuivre l'agitation pendant 15 minutes. Décanter, puis extraire la phase aqueuse par deux fois 100 ml d'éther éthylique. Laver les phases organiques réunies par deux fois 50 ml d'une solution saturée de chlorure d'ammonium puis sécher sur carbonate de sodium anhydre. Evaporer sous vide pour obtenir 27,8 g de produit brut. Après distillation à 138°C sous 0,3 mmHg, on obtient le (S) (N-benzyl N-éthyl amino)-2, (trifluorométhyl-3 phényl)-1 propane.

La pureté du produit est supérieure à 86%. Elle a été déterminée par chromatographie en phase gazeuse dans les conditions décrites à l'exemple 1.

$\alpha_D$ = +44,51

Rendement : 67,5% par rapport au (S) éthylbenzylamino-2, chloro-1 propane Spectre de résonance magnétique nucléaire ($^{13}C$) (T.M.S.) : 15,5 ppm ; 40 ppm ; 43 ppm ; 54 ppm ; 56 ppm ; 126-128-141-142 ppm.

**EXEMPLE 4 :**

**(S) éthylamino-2, (trifluorométhyl-3 phényl)-1 propane**

Dans une bombe d'hydrogénation, introduire 5 g de (S) (N-benzyl N-éthyl amino)-2, (trifluorométhyl-3 phényl)-1 propane avec 250 ml d'éthanol anhydre et 5 g de palladium à 5% sur charbon. Après avoir purgé la bombe à l'azote, agiter pendant 4 heures sous 10 bars d'hydrogène à +50°C. Ensuite éliminer le catalyseur par filtration et l'éthanol par évaporation et distiller le produit brut sous vide. On obtient 2,29 g de (S) éthylamino-2 (trifluorométhyl-3 phényl)-1 propane. Pureté 87,5%.

Rendement : 56% sur titre.

Spectre de résonance magnétique nucléaire ($^{13}C$) (T.M.S.) : 15,5 ppm ; 20,3 ppm ; 41.7 ppm ; 43.7 ppm ; 54,7 ppm ; 126-128-141 ppm.

## Revendications

1. Procédé de préparation énantiospécifique du (S) éthylamino-2 (trifluorométhyl-3 phényl)-1 propane de formule (I) :

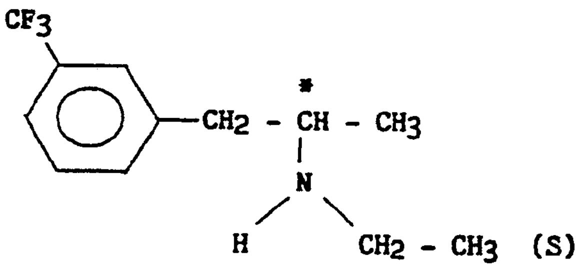

(I)

caractérisé en ce que :

— l'on condense le (S) alaninol, ou (S) amino-2 propanol-1, composé de formule II :

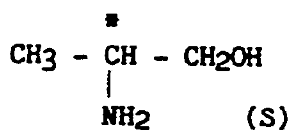

(II)

avec le benzaldéhyde, à température ambiante, et dans un solvant alcoolique anhydre, et ensuite, que l'on réduit à l'aide d'un borohydrure de métal alcalin pour obtenir le (S) N-benzyl alaninol ou (S) benzylamino-2 propanol-1 de formule III :

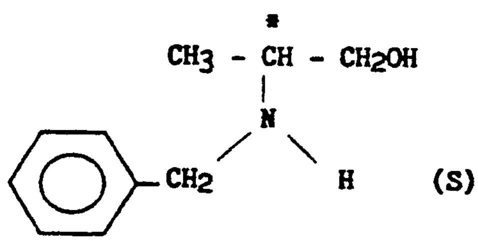

(III)

que l'on fait réagir avec l'acétaldéhyde dans le même milieu réactionnel, pour obtenir après réduction par un borohydrure de métal alcalin, le (S) N-benzyl, N-éthyl alaninol, ou (S) (N-benzyl N-éthyl amino)-2 propanol-1, composé de formule IV :

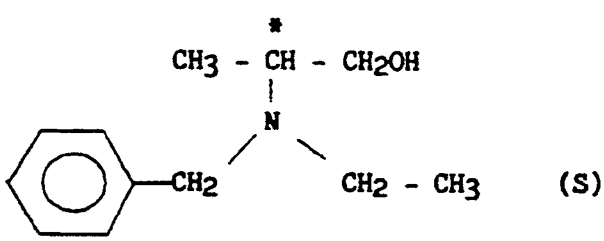

(IV)

que l'on soumet à l'action du chlorure de thionyle en solution dans l'éther et en présence d'un solvant aprotique, pour former le chlorhydrate du (S) (N-benzyl N-éthyl amino)-2 chloro-1 propane, composé de formule V :

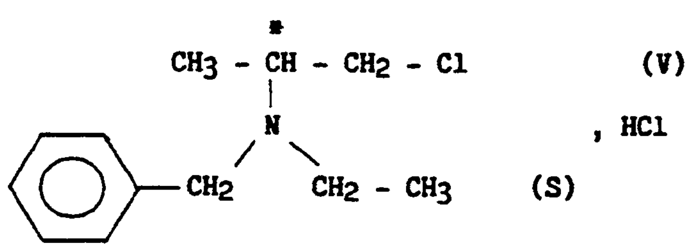

(V)

dont on libère la base par action d'un sel alcalin tel que le carbonate de sodium,

que l'on condense en présence d'un halogénure de cuivre, dans un solvant organique inerte, avec un composé organométallique de formule VI :

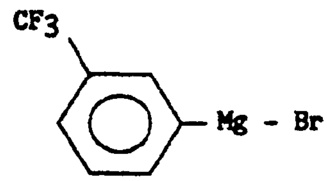

(VI)

pour former, après hydrolyse basique, le (S) (N-benzyl N-éthyl amino)-2 (trifluorométhyl-3 phényl)-1, propane composé de formule VII :

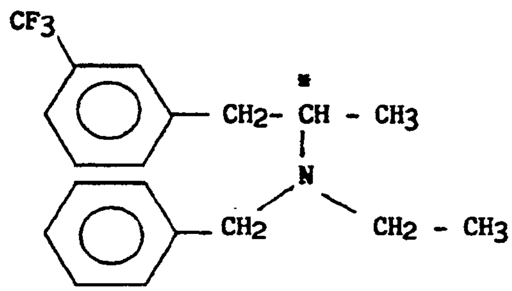

(VII)

que l'on soumet à une hydrogénation catalytique sous pression, en présence d'un métal de transition et à une température comprise entre 20° et 60°C pour obtenir le (S) éthylamino-2 (trifluorométhyl-3 phényl)-1 propane de formule I.

2. Procédé selon la revendication 1 caractérisé en ce que la condensation du (S) alaninol avec le benzaldéhyde et du N-benzyl alaninol avec l'acétaldéhyde est effectuée dans un solvant alcoolique, tel que le méthanol, et à une température comprise entre 0° et 20°C.

3. Procédé selon la revendication 1 caractérisé en ce que l'agent réducteur utilisé pour réduire le produit de condensation du (S) alaninol sur le benzaldéhyde et du N-benzyl alaninol sur l'acétaldéhyde est le borohydrure de sodium.

4. Procédé selon la revendication 1 caractérisé en ce que la réaction du chlorure de thionyle sur le N-benzyl N-éthyl, alaninol est effectuée dans l'éther éthylique, en présence d'hexaméthyle triamide phosphore.

5. Procédé selon la revendication 1 dans lequel le catalyseur utilisé pour condenser le composé VI avec le composé de formule V est le chlorure, le bromure ou l'iodure cuivreux.

6. Procédé selon la revendications 1 caractérisé en ce que le catalyseur utilisé pour l'hydrogénolyse du composé de formule VII est le palladium à 5% supporté sur charbon.

7. Procédé selon la revendication 1 caractérisé en ce que l'hydrogénolyse du composé du formule VII est effectuée sous une pression d'hydrogène comprise entre 5 et 15 bars, à une température comprise entre 20° et 60°C et dans un solvant alcoolique.

8. Le (S) (N-benzyl N-éthyl amino)-2 propanol-1 lorsque utilisé comme intermédiaire pour la préparation du (S) éthylamino-2 (trifluorométhyl-3 phényl)-1 propane selon le procédé de la revendication 1.

9. Le (S) (N-benzyl N-éthyl amino)-2 chloro-1 propane, lorsque utilisé comme intermédiaire pour la préparation du (S) éthylamino-2 (trifluorométhyl-3 phényl)-1 propane selon le procédé de la revendication 1.

10. Le (S) (N-benzyl N-éthyl amino)-2 (trifluorométhyl-3 phényl)-1 propane, lorsque utilisé comme intermédiaire pour la préparation du (S) éthylamino-2 (trifluorométhyl-3 phényl)-1 propane selon le procédé de la revendication 1.

**Patentansprüche**

1. Verfahren zur enantiospezifischen Herstellung von (S)-2-Ethylamino-1-(3-trifluormethyl-phenyl)-propan der Formel (I)

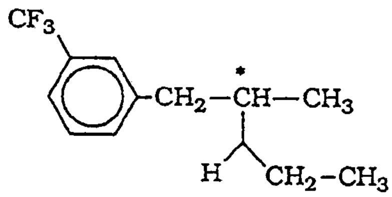

(I) (S)

**dadurch gekennzeichnet,** daß man

— (S)-Alaninol oder (S)-2-Amino-propanol-1 der Formel (II)

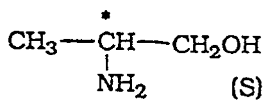

(II) (S)

mit Benzaldehyd bei Raumtemperatur in einem wasserfreien alkoholischen Lösungsmittel kondensiert und anschließend eine Reduktion mit einem Alkalimetallborhydrid durchführt zur Bildung von (S)-N-Benzyl-alaninol oder (S)-Benzyl-2-amino-propanol-1 der Formel (III)

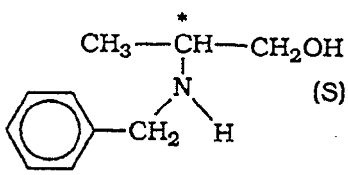

(III) (S)

welches man mit Acetaldeyhd in dem gleichen Reaktionsmedium umsetzt und mit einem Alkalimetallborhydrid reduziert zur Bildung von (S)-N-Benzyl, N-ethyl-alaninol oder (S)-2-(N-Benzyl,N-ethyl-amino)-propanol-1 der Formel (IV)

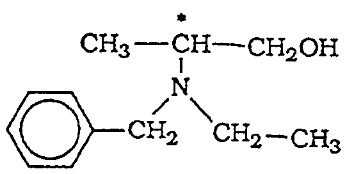

(IV) (S)

das man in Gegenwart eines aprotischen Lösungsmittels mit in Ether gelöstem Thionylchlorid umsetzt zur Bildung des Hydrochlorids von (S)-2-(N-Benzyl,N-ethyl-amino)-1-chlor-propan der Formel (V)

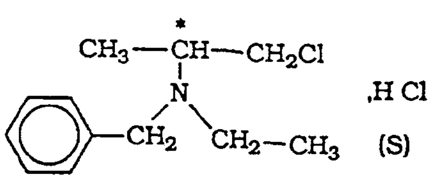

(V) ,H Cl (S)

aus dem man die Base durch Umsetzung mit einem alkalischen Salz, wie Natriumcarbonat, freisetzt, welche man in Gegenwart eines Kupferhalogenids in einem inerten organischen Lösungsmittel mit einer metallorganischen Verbindung der Formel (VI)

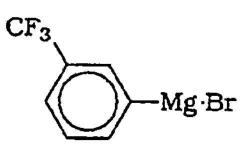

(VI)

umsetzt zur Bildung nach der basischen Hydrolyse von (S)-2-(N-Benzyl,N-ethyl-amino)-1-(3-trifluormethyl-phenyl)-propan der Formel (VII)

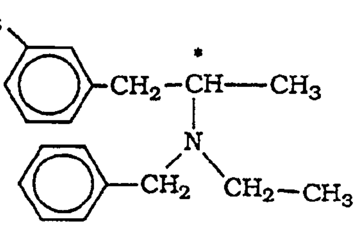

(VII)

welches man in Gegenwart eines Übergangsmetalls bei einer Temperatur zwischen 20°C und 60°C unter Druck katalytisch hydriert zur Bildung von (S)-2-Ethylamino-1-(3-trifluormethyl-phenyl)-propan der Formel (I).

2. Verfahren nach Anspruch 1, **dadurch gekennzeichnet,** daß die Kondensation von (S)-Alaninol mit Benzaldehyd und von N-Benzyl-alaninol mit Acetaldehyd in einem alkoholischen Lösungsmittel, wie Methanol, bei einer Temperatur zwischen 0°C und 20°C durchgeführt wird.

3. Verfahren nach Anspruch 1, **dadurch gekennzeichnet,** daß das zur Reduktion des Kondensationsprodukts von (S)-Alaninol mit Benzaldehyd und von N-Benzyl-alaninol mit Acetaldehyd verwendete Reduktionsmittel Natriumborhydrid ist.

4. Verfahren nach Anspruch 1, **dadurch gekennzeichnet,** daß die Umsetzung von Thionylchlorid mit N-Benzyl,N-ethyl-alaninol in Gegenwart von Hexamethylphosphorsäuretriamid in Ethylether durchgeführt wird.

5. Verfahren nach Anspruch 1, worin als Katalysator zur Kondensation der Verbindung der Formel (VI) mit der Verbindung der Formel (V) Kupfer(I)-chlorid, Kupfer(I)-bromid oder Küpfer(I)-iodid verwendet wird.

6. Verfahren nach Anspruch 1, **dadurch gekennzeichnet,** daß als Katalysator zur Hydrogenolyse der Verbindung der Formel (VII) 5% Palladium-auf-Kohlenstoff eingesetzt wird.

7. Verfahren nach Anspruch 1, **dadurch gekennzeichnet,** daß die Hydrogenolyse der Verbindung der Formel (VII) in einem alkoholischen Lösungsmittel bei einem Wasserstoffdruck zwischen 5 und 15 bar bei einer Temperatur zwischen 20°C und 60°C durchgeführt wird.

8. (S)-2-(N-Benzyl,N-ethyl-amino)-propanol-1 als Zwischenprodukt für die Herstellung von (S)-2-Ethylamino-1-(3-trifluormethyl-phenyl)-propan nach dem Verfahren gemäß Anspruch 1.

9. (S)-2-(N-Benzyl,N-ethyl-amino)-1-chlor-propan, als Zwischenprodukt für die Herstellung von (S)-2-Ethylamino-1-(3-trifluormethyl-phenyl)-propan nach dem Verfahren gemäß Anspruch 1.

10. (S)-2-(N-Benzyl,N-ethyl-amino)-1-(3-trifluormethyl-phenyl)-propan als Zwischenprodukt für die Herstellung von (S)-2-Ethylamino-1-(3-trifluormethyl-phenyl)-propan nach dem Verfahren gemäß Anspruch 1.

## Claims

1. Process for the enantiospecific preparation of (S)-2-ethylamino-1-(3-trifluoromethylphenyl)propane of the formula (I) :

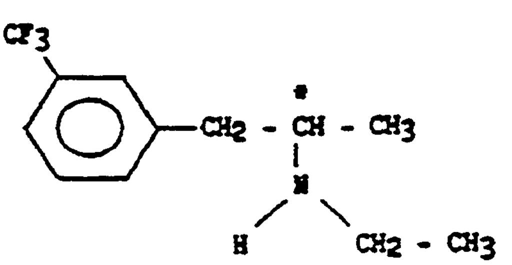

(S)

(I)

characterised in that :

— (S)-alaninol, or (S)-2-aminopropan-1-ol, compound of the formula II :

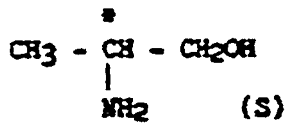

(II)

is condensed with benzaldehyde, at ambient temperature, and in an anhydrous alcoholic solvent, and then reduced by means of an alkali metal borohydride to obtain (S)-N-benzylalaninol or (S)-2-benzylaminopropan-1-ol of the formula III :

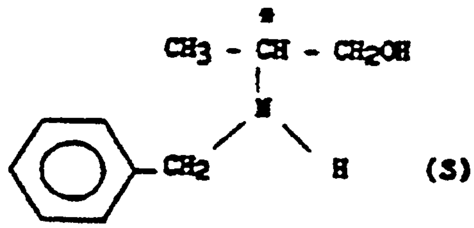

(III)

which is reacted with acetaldehyde in the same reaction medium, to obtain, after reduction by means of an alkali metal borohydride, (S)-N-benzyl-N-ethylalaninol, or (S)-2-(N-benzyl-N-ethylamino)propan-1-ol, compound of the formula IV :

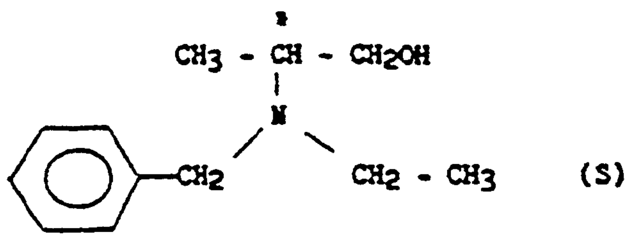

(IV)

which is subjected to the action of thionyl chloride in solution in ether and in the presence of an aprotic solvent, to form (S)-2-(N-benzyl-N-ethylamino)-1-chloropropane hydrochloride, compound of the formula V :

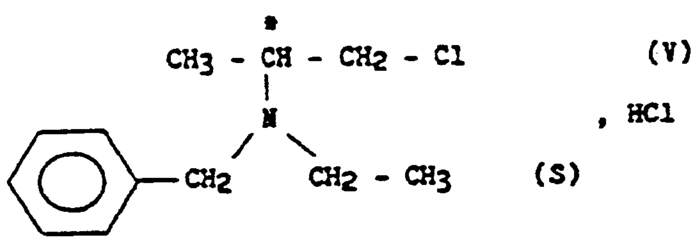

(V)

the base of which is freed by the action of an alkaline salt such as sodium carbonate,
and which is condensed in the presence of a copper halide, in an inert organic solvent, with an organometallic compound of the formula VI :

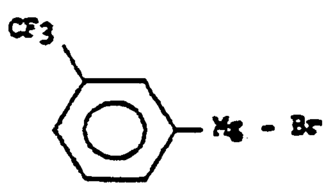

(VI)

to form, after basic hydrolysis, (S)-2-(N-benzyl-N-ethylamino)-1-(3-trifluoromethylphenyl)propane, compound of the formula VII :

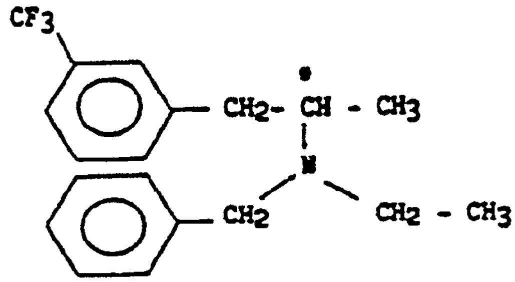

(VII)

which is subjected to catalytic hydrogenation under pressure, in the presence of a transition metal and at a temperature ranging between 20° and 60°C to obtain (S)-2-ethylamino-1-(3-trifluoromethylphenyl)propane of the formula I.

2. Process according to Claim 1, characterised in that the condensation of (S)-alaninol with benzaldehyde and of N-benzylalaninol with acetaldehyde is carried out in an alcoholic solvent, such as methanol, and at a temperature ranging between 0° and 20°C.

3. Process according to Claim 1, characterised in that the reducing agent used to reduce the condensation product of (S)-alaninol with benzaldehyde and of N-benzyl-alaninol with acetaldehyde is sodium borohydride.

4. Process according to Claim 1, characterised in that the reaction of thionyl chloride with N-benzyl-N-ethylalaninol is carried out in diethyl ether, in the presence of hexamethyl phosphoric triamide.

5. Process according to Claim 1, wherein the catalyst used to condense the compound VI with the compound of formula V is cuprous chloride, bromide or iodide.

6. Process according to Claim 1, characterised in that the catalyst used for the hydrogenolysis of the compound of formula VII is 5% palladium supported on charcoal.

7. Process according to Claim 1, characterised in that the hydrogenolysis of the compound of formula VII is carried out under a hydrogen pressure ranging between 5 and 15 bars, at a temperature ranging between 20° and 60°C and in an alcoholic solvent.

8. (S)-2-(N-benzyl-N-ethylamino)propan-1-ol when used as an intermediate product for the preparation of (S)-2-ethylamino-1-(3-trifluoromethylphenyl)propane according to the process of Claim 1.

9. (S)-2-(N-benzyl-N-ethylamino)-1-chloropropane, when used as an intermediate product for the preparation of (S)-2-ethylamino-1-(3-trifluoromethylphenyl)propane according to the process of Claim 1.

10. (S)-2-(N-benzyl-N-ethylamino)-1-(3-trifluoromethylphenyl)propane, when used as an intermediate product for the preparation of (S)-2-ethylamino-1-(3-trifluoromethylphenyl)propane according to the process of Claim 1.